**Europäisches Patentamt**

**European Patent Office**

**Office européen des brevets**

(19)

(11) Veröffentlichungsnummer: **0 143 411**
**B1**

## (12) EUROPÄISCHE PATENTSCHRIFT

(45) Veröffentlichungstag der Patentschrift:
26.07.89

(51) Int. Cl.⁴: **G 06 F 15/02**, G 06 F 15/42

(21) Anmeldenummer: **84113939.7**

(22) Anmeldetag: **17.11.84**

(54) **Uhr mit Vorrichtung zur Ermittlung des Blutalkoholgehaltes.**

(30) Priorität: **22.11.83 DE 3342100**

(43) Veröffentlichungstag der Anmeldung:
**05.06.85 Patentblatt 85/23**

(45) Bekanntmachung des Hinweises auf die Patenterteilung:
**26.07.89 Patentblatt 89/30**

(84) Benannte Vertragsstaaten:
**AT BE CH DE FR GB IT LI NL SE**

(56) Entgegenhaltungen:
**US-A- 3 668 866**
**US-A- 4 380 802**

**TECHNISCHE RUNDSCHAU, Band 70, Nr. 14, April 1978,
Seiten 20-25, Berlin, DE; Dr. W. HÜRLIMANN:
"Rechnende Uhren"**

(73) Patentinhaber: **Coeppicus, Rolf, Nordstrasse 17,
D-4200 Oberhausen 14 (DE)**

(72) Erfinder: **Coeppicus, Rolf, Nordstrasse 17,
D-4200 Oberhausen 14 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zur elektronischen Ermittlung der Alkoholkonzentration im Blut.

Bekannt sind Vorrichtungen, bei denen der Blutalkoholgehalt auf chemischem Wege analysiert und elektronisch angezeigt wird. Diese Vorrichtungen haben den Nachteil, daß sie verhältnismäßig aufwendig konstruiert und von daher für den Allgemeingebrauch ungeeignet sind. Nachteilig ist vor allem, daß dabei Blut entnommen und überprüft werden muß.

Andererseits ergibt sich der Promillewert der Alkoholkonzentration im Blut, der durch das Trinken von Alkohol oder alkoholischen Getränken entsteht, nicht zufällig, sondern gesetzmäßig. Die Höhe der Alkoholkonzentration im Blut (BAK) ist abhängig von:

- der Menge des getrunkenen Alkohols,
- der Trinkgeschwindigkeit,
- dem Körpergewicht und
- der Trinkzeit.

Je mehr Alkohol jemand trinkt, umso stärker steigt seine BAK an. Es besteht eine strenge Proportionalität zwischen der Trinkmenge und der Blutalkoholkonzentration.

Mit dem ersten Schluck beginnt die Aufnahme des Alkohols in das Blut (Resorption). Der getrunkene Alkohol wird aber nicht auf einmal resorbiert. Ein Glas Schnaps ist nach ca. fünf Minuten, ein Glas Bier nach ca. zehn Minuten resorbiert. Trinkt jemand vier Gläser in zeitlich kurzem Abstand, so wird der in diesen vier Bier enthaltene Alkohol nicht in zehn Minuten, sondern in ca. 40 Minuten resorbiert. Die Resorption wird also vom Trinktempo nicht beeinträchtigt, insbesondere nicht beschleunigt.

Die getrunkene Alkoholmenge verdünnt sich in einer großen Körpermasse stärker als in einer kleinen Körpermasse. Die Blutalkoholkonzentration ist bei gleicher Trinkmenge dem Körpergewicht umgekehrt proportional. So ist bei gleicher Trinkmenge der Promillegehalt bei 80 Kg Körpergewicht niedriger als bei 70 Kg Körpergewicht.

Mit dem Übergang des Alkohols aus dem Magen in den menschlichen Körper beginnt gleichzeitig der Abbau (die Verbrennung) des Alkohols. Der Abbau des Alkohols hat ständig die gleiche Höhe (PONSSOLD). Die Höhe des Abbaus hängt vom Körpergewicht ab und beträgt bei einem Körpergewicht von 60 Kg ca. 6,6 g/Std., bei einem Körpergewicht von 80 Kg etwa 8,8 g/Std. Diesem unterschiedlichen Abbau in Gramm bei unterschiedlichen Körpergewichten entspricht aber trotzdem auch bei unterschiedlichen Körpergewichten demselben Promillewert von etwa 0,16 Promille und entspricht grob einer Trinkeinheit. Wer also zu Beginn einer jeden Stunde ein Glas Bier trinkt, dessen Blutalkoholkonzentration ist am Ende einer jeden Stunde wieder auf Null Promille gefallen. Das entspricht der Erfahrung, die jeder schon gemacht hat. Aus der US-A-4 380 802 ist eine Vorrichtung zur Ermittlung der von einer Person aufgenommenen Kalorien, der von einer Person verbrannten Kalorien und der dazwischenliegenden Differenz bekannt. Die bekannte Vorrichtung weist eine elektronische Auswertevorrichtung mit einer digitalen Anzeige und verschiedene Tasten auf. Zusätzliche Steuerschalter dienen der Auswahl der verschiedenen möglichen Berechnungen.

Ausgehend von diesen Erkenntnissen liegt der Erfindung die Aufgabe zugrunde, eine für den Laien einfach zu handhabende und betätigungssichere Promillekontrolleinrichtung zu schaffen.

Die Aufgabe wird erfindungsgemäß dadurch gelöst, daß einer elektronischen Auswerteeinrichtung mit einer digitalen Anzeige Eingabetasten für das Körpergewicht, Eingabetasten für mehrere unterschiedliche alkoholische Getränke bzw. Getränkemengen sowie eine Ergebnistaste zugeordnet sind, wobei bei Betätigung der letzteren Tasten gleichzeitig ein der elektronischen Auswerteeinrichtung zugeordneten Zeitmesser in Betrieb gesetzt wird, welcher die notwendigen Zeitdaten zur Ermittlung der Alkoholkonzentration im Blut liefert.

Derartige Auswerteeinrichtungen können durch einen Mikroprozessor dargestellt sein. Dieser Mikroprozessor errechnet nach Eingabe der Trinkmenge unter Berücksichtigung des Körpergewichtes und der abgelaufenen Zeit den Alkoholaufbau und gleichzeitig auch den Alkoholabbau im Blut. Für die Eingabe der unterschiedlichen alkoholischen Getränkesorten und Getränkemengen hat die Vorrichtung verschiedene Eingabetasten. Diese Eingabetasten sind ebenso zusammengefaßt, wie die Eingabetasten für das Körpergewicht. Die Ergebnistaste, mit der das jeweilige Ergebnis abgerufen werden kann, ist zweckmäßig neben den Eingabetasten und getrennt dazu angeordnet. Das Ergebnis erscheint bei Betätigen der Ergebnistaste in der entsprechenden digitalen Anzeige.

Die geschilderte elektronische Auswerteeinrichtung ist vorab mit Durchschnitts- und Extremwerten für den Reduktionsfaktor, Wert für den Alkoholabbau, Kontrollfaktor für die Übernahme des Alkohols in das Blut bzw. Gewebe und Ablauf der Resorption gespeist. Um dem Benutzer eine Kontrolle zu ermöglichen und andererseits die Warnfunktion zu verbessern, ist nach Ausbildung der Erfindung eine der Ergebnistaste parallel geschaltete Sicherheitstaste vorgesehen. Über diese Sicherheitstaste werden erfindungsgemäß die jeweils gespeicherten ungünstigsten Fixfaktoren aktiviert, so daß der über die Sicherheitstaste ermittelte Wert der Blutalkoholkonzentration den für den jeweiligen Benutzer ungünstigsten und damit sichersten Wert in der digitalen Anzeige angezeigt wird.

Eine besonders zweckmäßige und für den Gebrauch optimale Ausbildung ist die, bei der die Auswerteeinrichtung sowie die Anzeige, Eingabe- und Ergebnistaste sowie Sicherheitstaste in eine elektronische Uhr, vorzugsweise Armbanduhr integriert sind. Eine derartige mit der üblichen Zeitanzeige verbundene Armbanduhr wird in der Regel immer mitgetragen, so daß sie in allen Zweifelsfällen eingeschaltet und benutzt werden kann.

Hierdurch ist sichergestellt, daß der Benutzer auch in nicht eingeplanten Fällen immer sicher seinen Blutalkoholwert ermitteln kann.

Um zu verhindern, daß unbeabsichtigt Werte eingegeben ggf. sogar falsche Werte eingegeben werden, weisen die Eingabetasten eine flächenmäßig begrenzte oder geschützt ausgebildete Berührungsfläche auf. Damit können die einzelnen Eingabetasten nur mit Absicht betätigt werden, wobei indirekt dadurch sichergestellt ist, daß bei überhöhtem Alkoholgenuß eine unrichtige Betätigung der Promilleuhr nicht mehr möglich ist. Damit ist eine mißbräuchliche Benutzung annähernd ausgeschlossen, was noch dadurch verbessert werden kann, daß bei Erreichen eines Promillewertes von über 0,8 beispielsweise ein weiterer Betrieb der Promilleuhr nur durch Betätigung einer zusätzlichen Kontrolltastatur möglich ist. Die sichere Ausbildung der Eingabetasten ist insbesondere dann gewährt, wenn die Berührungsfläche auf die Kugelschreiberminenspitze zugeschnitten ist.

Da auch die Zeit einen wesentlichen Faktor bei der Berechnung des Blutalkoholgehaltes darstellt, ist eine entsprechende Eingabe notwendig. Eine gesonderte Eingabetaste erübrigt sich erfindungsgemäß, da die Eingabetasten, vorzugsweise die Eingabetasten für die alkoholischen Getränke, mit einem der elektronischen Auswerteeinheit zugeordneten Zeitmesser gekoppelt sind. Damit wird die Uhrzeit automatisch berücksichtigt, wodurch Fehler auf einfache Art und Weise vermieden sind. Über die Ergebnistaste kann der Zeitmesser ausgeschaltet werden, wobei allerdings aus Sicherheitsgründen hier ebenfalls eine zusätzliche Taste vorzusehen ist, über die der Zeitmesser entweder gezielt ausgeschaltet oder aber bei Nichtbetätigen in einem gewissen Zeitraum automatisch ausgeschaltet wird.

Die Erfindung zeichnet sich insbesondere dadurch aus, daß eine für den Laien einfach zu betätigende Vorrichtung zur elektronischen Ermittlung der Alkoholkonzentration im Blut geschaffen ist, die durch die Integration in eine übliche elektronische Armbanduhr so untergebracht ist, daß sie im Bedarfsfall immer greifbar ist. Durch geschickte Ausbildung und Anordnung der Eingabetasten und entsprechende Schaltung ist die Eingabe von Daten auf eine Minimalzahl begrenzt, während übrige für das Berechnungsverfahren benötigte Daten im Durchschnittswert Berücksichtigung finden.

Weitere Einzelheiten und Vorteile des Erfindungsgegenstandes ergeben sich aus der nachfolgenden Beschreibung der zugehörigen Zeichnung, in der ein bevorzugtes Ausführungsbeispiel mit den dazu notwendigen Einzelheiten und Einzelteilen dargestellt ist. Es zeigen:

Fig. 1 eine Promilleuhr in Draufsicht und

Fig. 2 ein vereinfachtes Schaltbild des die Promilleermittlung betreffenden Teils der Uhr nach Fig. 1.

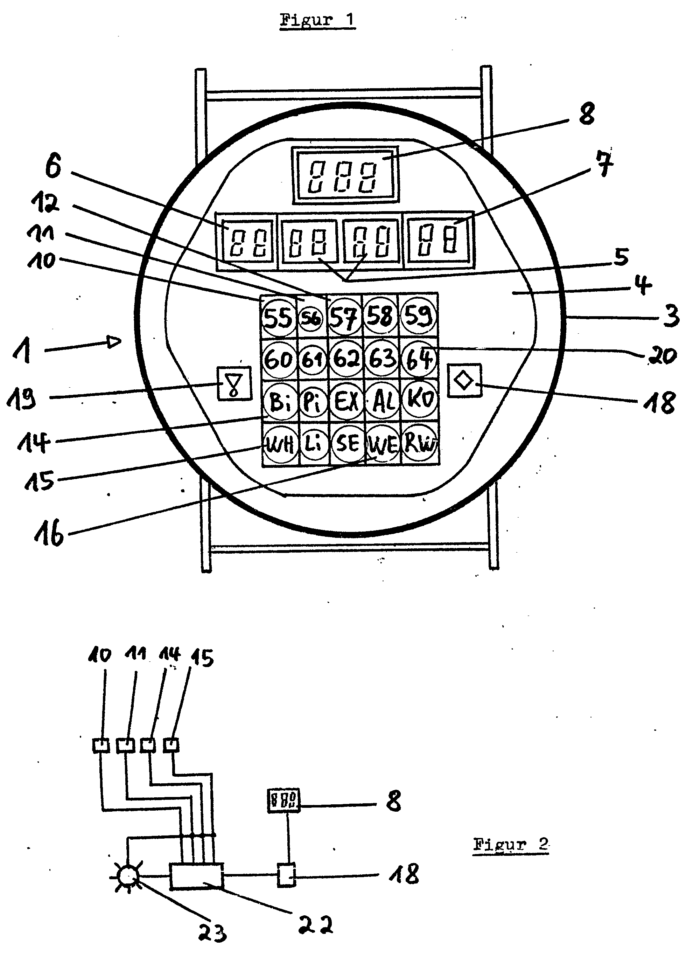

Die in Fig. 1 dargestellte Promilleuhr 1 ist in eine übliche elektronische Armbanduhr 3 integriert bzw. als solche ausgebildet. Das Ziffernblatt 4 weist zusätzlich zur Anzeige 5 der Uhrzeit sowie der Anzeige 6 des Tages bzw. Monates und der Anzeige 7 mit Terminvorgaben eine Anzeige 8 für Promillewerte auf. Diese Anzeigen 5, 6, 7, 8 sind so angeordnet, daß die Anzeige 8 für die Promille gesondert angeordnet und als solche kenntlich gemacht ist.

Darunter befindet sich ein großes Feld mit Eingabetasten 10, 11, 12 für das Körpergewicht und Eingabetasten 14, 15, 16 für Getränkearten und/oder Getränkemengen. Rechts neben diesem Block ist die Ergebnistaste 18 als solche kenntlich gemacht angeordnet, während auf der gegenüberliegenden Seite die Sicherheitstaste 19 vorgesehen ist, die ebenfalls als solche kenntlich gemacht und damit eigentlich nur getrennt von der Ergebnistaste 18 betätigt werden kann.

Die Berührungsflächen 20 der einzelnen Eingabetasten 10, 11, 12, 13, 14, 15, 16 sind so bemessen, daß sie nur mit Hilfe der Kugelschreiberminenspitze betätigt werden können. Weitere Möglichkeiten der Sicherung sind die der versteckten Anordnung der Tasten oder auch der Sicherung über einen Schiebekontakt.

Fig. 2 zeigt das schematisierte Schaltbild, wobei die Auswerteeinrichtung mit 22 bezeichnet ist. Die Auswerteeinheit 22 ist über die einzelnen Eingabetasten 10, 11 bzw. 14, 15 ansprechbar, wobei diese bei Betätigung gleichzeitig auch den Zeitmesser 23 in Betrieb setzen, der die notwendigen Zeitdaten liefert. Bei Betätigen der Ergebnistaste 18 erscheint dann das Auswerteergebnis auf der Anzeige 8.

Zur Handhabung der Promilleuhr 1 ist zu sagen, daß eine Taste für den Trinkbeginn wie erwähnt unnötig ist, da die Zeitmessung mit der Eingabe des genossenen Alkohols automatisch beginnt. Der Autofahrer bzw. Benutzer gibt bei Beginn des Trinkens sein Körpergewicht ein. Danach gibt er durch Drücken der Eingabetasten die jeweils getrunkene Alkoholmenge ein, wobei er aus Sicherheitsgründen die Uhr bzw. die entsprechende Eingabetaste in dem Augenblick bedient, wenn das neue, volle Glas auf den Tisch gestellt wird. Damit wird gleichzeitig der zeitmessende Teil der Uhr in Gang gesetzt, der entsprechend dem Körpergewicht und der ablaufenden Zeit den Aufbau des Blutalkoholgehaltes, aber auch den Abbau mißt. Will der Benutzer seinen derzeitigen Blutalkoholgehalt wissen, drückt er auf die Ergebnistaste 18, die ihm den aufgebauten, abzüglich des abgebauten Alkohols im Blut und somit seinen derzeitigen Blutalkoholgehalt in Promille auf der Anzeige 8 ablesbar anzeigt.

Für die Eingabe der unterschiedlichen alkoholischen Getränkesorten und Mengen sind unterschiedliche Eingabetasten 14, 15, 16 beispielsweise für Bier, Alt, Wein, Korn, Whisky, Likör u.ä. vorgesehen. Bei den Eingabetasten 10, 11, 12 für das Körpergewicht handelt es sich um verhältnismäßig niedrige Angaben, was darauf zurückzuführen ist, daß die hier abgebildete Uhr offenbar für Damen vorgesehen ist.

Die Blutalkoholkonzentration, die nach dem Trinken einer bestimmten Menge Alkohols eintreten wird, errechnet sich aus einer Formel, bei der auch der Reduktionsfaktor eine wesentliche Rolle spielt. Dieser besagt, daß der Alkohol nicht nur vom Blut, sondern auch vom sonstigen Körpergewebe aufgenommen wird. Will man also aus der Menge des getrunkenen Alkohols die Blutalkoholkonzentration errechnen, muß man den Alkohol, der nicht vom Blut, sondern vom sonstigen Körper aufgenommen wird, abziehen. Denn durch die Blutentnahme wird nur die Alkoholkonzentration im Blut gemessen. Das geschieht durch den Reduktionsfaktor. Die mittlere Alkoholkonzentration im Gesamtkörper verhält sich zur Alkoholkonzentration im Blut wie 0,7 : 1, so daß der Reduktionsfaktor bei etwa 0,7 liegt. Bei Pyknikern und Frauen kann er bei 0,6, bei Asthenikern bei 0,8 liegen.

Über die Ergebnistaste 18 errechnet die Auswerteeinrichtung 22 die Blutalkoholkonzentration wie geschildert unter Anwendung des Reduktionsfaktors 0,7. Die Ergebnistaste 18 ruft in der Auswerteeinrichtung auch bei den übrigen Faktoren die Normaldaten ab. Sie kommt damit zu einer Blutalkoholkonzentration, wie sie ohne das Hinzutreten ungünstiger Umstände üblicherweise vorliegt.

Beim Vorliegen besonderer Umstände kann der Promillewert höher liegen, als bei Berücksichtigung der Normalwerte. So wird durch eine kurz vor dem Trinken eingenommene Mahlzeit über 30% der getrunkenen Alkoholmenge vom Blut nicht übernommen, während beim Trinken auf nüchternen Magen nur eine Menge von ca. 10% des getrunkenen Alkohols nicht resorbiert wird. Weiter ist der Alkoholabbau unterschiedlich. Der durchschnittliche Abbauwert liegt bei 0,14 Promille je Stunde, kann aber auch 0,21 Promille/Stunde betragen.

Über die Sicherheitstaste 19 berücksichtigt die Auswerteeinrichtung alle Faktoren, die im angenommenen ungünstigsten Fall zusammentreffen und den Promillewert erhöhen können. Über die Sicherheitstaste 19 werden somit in der Auswerteeinrichtung 22 die entsprechenden ungünstigen Faktoren abgerufen. So wird bei Benutzung der Sicherheitstaste ein Reduktionsfaktor von 0,6 berücksichtigt, es wird davon ausgegangen, daß der Benutzer vor und während des Trinkens nichts ißt, daß die Blutalkoholkonzentration wegen nicht vollendeter Resorption noch steigen wird und daß lediglich ein Abbauwert von 0,10 Promille für jede seit Trinkbeginn verstrichene Stunde anzusetzen ist. Der beim Drücken der Sicherheitstaste in der Anzeige 8 wiedergegebene Wert wird also immer größer oder mindestens gleich groß sein, wie der Wert einer zur gleichen Zeit entnommenen Blutprobe. Der Benutzer kann also sicher sein, daß bei Entnahme einer Blutprobe die durch die Sicherheitstaste angezeigte Blutalkoholkonzentration nicht überschritten wird.

Zur Kontrolle der Richtigkeit der Eingabe erscheint die Eingabe sichtbar auf der Anzeigeeinrichtung, z.B. «Pils 0,2 l». Wenn der Benutzer das liest, dann weiß er, daß dieses Glas Pils bei der zu errechnenden Blutalkoholkonzentration berücksichtigt wird.

## Patentansprüche

1. Vorrichtung zur elektronischen Ermittlung der Alkoholkonzentration im Blut, dadurch gekennzeichnet, daß einer elektronischen Auswerteeinrichtung (22) mit einer digitalen Anzeige (5, 6, 7, 8) Eingabetasten (10, 11, 12) für das Körpergewicht, Eingabetasten (14, 15, 16) für mehrere unterschiedliche alkoholische Getränke bzw. Getränkemengen sowie eine Ergebnistaste (18) zugeordnet sind, wobei bei Betätigung der Eingabetasten (14, 15, 16) für Getränke bzw. Getränkemengen gleichzeitig ein der elektronischen Auswerteeinrichtung (22) zugeordneter Zeitmesser (23) in Betrieb gesetzt wird, welcher die notwendigen Zeitdaten zur Ermittlung der Alkoholkonzentration im Blut liefert.

2. Vorrichtung nach Anspruch 1, dadurch gekennzeichnet, daß eine der Ergebnistaste (18) parallel geschaltete Sicherheitstaste (19) vorgesehen ist.

3. Vorrichtung nach Anspruch 1 und Anspruch 2, dadurch gekennzeichnet, daß die Auswerteeinrichtung (22) sowie die Anzeige (5, 6, 7, 8), Eingabe- (10, 11, 12, 14, 15, 16) und Ergebnistaste (18) sowie Sicherheitstaste (19) in eine elektronische Uhr vorzugsweise Armbanduhr (3) integriert sind.

4. Vorrichtung nach Anspruch 1, dadurch gekennzeichnet, daß die Eingabetasten (10, 11, 12, 14, 15, 16) eine flächenmäßig begrenzte oder geschützt ausgebildete Berührungsfläche (20) aufweisen.

5. Vorrichtung nach Anspruch 4, dadurch gekennzeichnet, daß die Berührungsfläche (20) auf die Kugelschreiberminenspitze zugeschnitten ist.

6. Vorrichtung nach Anspruch 2, dadurch gekennzeichnet, daß über die Sicherheitstaste (19) die jeweils gespeicherten ungünstigsten Fixfaktoren aktivierbar sind.

7. Vorrichtung nach Anspruch 1, dadurch gekennzeichnet, daß beim Drücken der Eingabetasten (14, 15, 16) das eingegebene alkoholische Getränk nach Art und Menge in der digitalen Anzeige (8) sichtbar wird.

## Claims

1. Device for electronically determining the alcohol concentration in the blood, characterized in that input buttons (10, 11, 12) for the body weight, input buttons (14, 15, 16) for a plurality of different alcoholic beverages or beverage quantities as well as a result button (18) are associated with an electronic evaluating device (22) having a digital display (5, 6, 7, 8), wherein with the actuation of the input buttons (14, 15, 16) for beverages or beverage quantities a timer associated with the electronic evaluating device (23) is simultaneously activated, which provides the necessary time data for determining the alcohol concentration in the blood.

2. Device according to claim 1, characterized in that there is provided a safety button (19) arranged in parallel with the result button (18).

3. Device according to claim 1 and claim 2, characterized in that the evaluating device (22) as well as the display (5, 6, 7, 8), the input- (10, 11, 12, 14, 15, 16) and result button (18) as well as the safety button (19) are integrated in an electronic watch preferably a wrist watch (3).

4. Device according to claim 1, characterized in that the input buttons (10, 11, 12, 14, 15, 16) are provided with a contact surface (20) of limited or protected area.

5. Device according to claim 4, characterized in that the contact surface (20) is shaped in adaption of the ball-point pen refill tip.

6. Device according to claim 2, characterized in that by means of the safety button (19) the stored worsest constant factors are adapted to be activated.

7. Device according to claim 1, characterized in that upon actuation of the input buttons (14, 15, 16) the entered alcoholic beverage is displayed according to type and quantity in the digital display (8).

**Revendications**

1. Dispositif de détermination électronique de la concentration d'alcool dans le sang, caractérisé en ce qu'il comporte un moyen d'évaluation électronique (22) avec un affichage digital (5, 6, 7, 8), des touches d'entrée (10, 11, 12) pour le poids du corps, des touches d'entrée (14, 15, 16) pour plusieurs boissons ou quantités de boisson alcoolisées différentes ainsi qu'une touche de résultat (18), par lequel, lors de la commande des touches d'entrée (14, 15, 16) pour boissons ou quantité de boisson, un chronomètre (23) adjoint au moyen d'évaluation électronique (22) est mis en marche simultanément, ce chronomètre fournissant les données de temps nécessaires pour la détermination de la concentration d'alcool dans le sang.

2. Dispositif selon la revendication 1, caractérisé en ce qu'une touche de sécurité (19) montée parallèlement à la touche de résultat (18) est pourvue.

3. Dispositif selon la revendication 1 et la revendication 2, caractérisé en ce que le moyen d'évaluation électronique (22) ainsi que l'affichage (5, 6, 7, 8), les touches d'entrée (10, 11, 12, 14, 15, 16) et la touche de résultat (18) ainsi que la touche de sécurité (19) sont intégrées dans une montre électronique, de préférence une montre-bracelet (3).

4. Dispositif selon la revendication 1, caractérisé en ce que les touches d'entrée (10, 11, 12, 14, 15, 16) présentent une zone de contact (20) limitée superficiellement ou conformée de façon protégée.

5. Dispositif selon la revendication 4, caractérisé en ce que la zone de contact (20) est taillée aux dimensions de la pointe d'un stylo à bille.

6. Dispositif selon la revendication 2, caractérisé en ce que les facteurs constants les plus défavorables emmagasinés momentanément sont rendus actifs au moyen de la touche de sécurité (19).

7. Dispositif selon la revendication 1, caractérisé en ce que, lors de la pression des touches d'entrée (14, 15, 16), la boisson alcoolisée donnée est visible selon sa sorte et sa quantité dans l'affichage digital (8).

Figur 1

Figur 2